# EUROPÄISCHE PATENTSCHRIFT

(11) **EP 1 385 568 B1**
(45) Veröffentlichungstag und Bekanntmachung des Hinweises auf die Patenterteilung: **18.04.2007**
(21) Anmeldenummer: 02730234.8
(22) Anmeldetag: 03.05.2002
(51) Int. Cl.: A61M 21/00

(54) **KISSEN, INSBESONDERE FÜR DEN EINSATZ IM RAHMEN VON THERAPEUTISCHEN MASSNAHMEN**
PILLOW, ESPECIALLY FOR USE DURING THERAPEUTIC MEASURES
OREILLER, CONÇU EN PARTICULIER POUR ETRE UTILISE DANS LE CADRE DE MESURES THERAPEUTIQUES

(30) Priorität: 07.05.2001 DE 20107734 U
(43) Veröffentlichungstag der Anmeldung: 04.02.2004
(73) Patentinhaber: Göhl, Hartmut E., 41564 Kaarst (DE)
(72) Erfinder: Göhl, Hartmut E., 41564 Kaarst (DE)
(74) Vertreter: Paul, Dieter-Alfred
(86) Internationale Anmeldenummer: PCT/EP2002/004885
(87) Internationale Veröffentlichungsnummer: WO 2002/089905

(56) Entgegenhaltungen:
- WO-A-99/21460
- DE-U- 9 410 690
- US-A- 2 958 769
- US-A- 3 290 450
- US-A- 4 782 533
- US-A- 5 479 667

## Beschreibung

Die vorliegende Erfindung betrifft ein Kissen, insbesondere für den Einsatz im Rahmen von therapeutischen Maßnahmen, mit einem Kissenkörper aus einem elastisch verformbaren Material, in dem mehrere Lautsprecher positionierbar sind und der an seiner Oberseite eine Auflagefläche bildet.

Kissen dieser Art mit eingebauten Lautsprechern sind aus der WO 99/58088 bekannt und werden in erster Linie eingesetzt, um Ohrerkrankungen wie beispielsweise Tinnitus Aurium durch sogenannte Musiktherapien zu behandeln, wobei den Tinnitus-Erkrankten entsprechend der Gehörstörung Musikstücke vorgespielt werden, welche die von dem Tinnitus-Erkrankten subjektiv empfundenen Ohrgeräusche überlagern.

Der Einsatz solcher musiktherapeutischer Kissen hat sich im Rahmen dieser Behandlung von Tinnitus-Erkrankungen oder anderen Schlafstörungen durchaus bewährt. Als nachteilig wird jedoch empfunden, daß die Kissen entweder gar nicht oder nur nach aufwendiger Demontage der Komponenten des Lautsprechersystems gereinigt werden können und außerdem auch die individuelle Positionierung der Lautsprecher an die Wünsche eines Patienten mit erheblichem Aufwand verbunden ist.

In der US 5,479,667 ist ein Kissen beschrieben, das für therapeutische Maßnahmen eingesetzt wird. In einem Kissenkörper, der aus einem elastischen verformbaren Material besteht, sind mehrere Lautsprecher positionierbar. Die Oberseite des Kissenkörpers bildet eine Auflagefläche.

Aufgabe der Erfindung ist es, ein Kissen der eingangs genannten Art so auszugestalten, daß das Kissen leicht gereinigt werden kann und insbesondere die Lautsprecher auch in einfacher Weise positioniert werden können.

Diese Aufgabe ist erfindungsgemäß dadurch gelöst, daß das Kissen eine Schiene aus einem elastischen verformbaren Material enthält, die in eine Korrespondierende Ausnehmung an der Unterseite des Kissenkörpers lösbar einsetzbar ist, wobei die Schiene so ausgebildet ist, daß Lautsprecher in der Schiene einsetzbar sind.

Erfindungsgemäß sind die Lautsprecher somit nicht mehr wie im Stand der Technik direkt in den Kissenkörper eingesetzt, sondern an einer Schiene vormontiert und können somit auf einfache Weise an dem Kissenkörper angebracht oder zur Reinigung des Kissenkörpers von diesem gelöst werden, indem die Schiene in die hierzu vorgesehene Ausnehmung an der Kissenkörperunterseite eingesetzt oder daraus wieder entfernt wird.

Zweckmäßigerweise weist die Schiene Aussparungen insbesondere in Form von Durchgangsöffnungen, die sich besonders leicht herstellen lassen, auf, in welche die Lautsprecher einsetzbar sind. Gemäß einer bevorzugten Ausführungsform sind die Lautsprecheraussparungen in den axialen Endbereichen der Schiene vorgesehen, so daß die darin positionierten Lautsprecher möglichst weit auseinanderliegen und insbesondere beidseitig eines auf dem Kissen ruhenden Kopfes plaziert sind. Hierdurch läßt sich die bestmögliche Klangwirkung erzielen.

Wenn der Kissenkörper orthopädisch geformt ist, kann es auch sinnvoll sein, die Ausnehmung für die Schiene im Nackenbereich des Kissens, wo sich üblicherweise eine Nackenwulst befindet, an der Kissenunterseite vorzusehen. Der Patient hört dann nicht nur über die Ohren, sondern auch über die sogenannten Hörknochen.

Gemäß einer bevorzugten Ausführungsform der Erfindung ist vorgesehen, daß der Kissenkörper und die Schiene jeweils aus. Schaumstoffmaterial bestehen, so daß die Schiene leicht in die korrespondierende Ausnehmung des Kissenkörpers eingesetzt und wieder aus dieser entfernt werden kann. Bei entsprechender Dimensionierung hält die Schiene in der Ausnehmung dann von selbst, so daß keine zusätzlichen Befestigungselemente wie beispielsweise Klettbänder oder dergleichen zur Anbringung der Schiene an dem Kissenkörper erforderlich sind. In gleicher Weise lassen sich die Lautsprecher in den Aussparungen leicht und sicher positionieren. Es hat sich als vorteilhaft erwiesen, wenn die Schiene hierbei zumindest großenteils aus einem Hartschaummaterial besteht, wodurch der Schiene eine gewisse Festigkeit gegeben wird. Um hierdurch die Bequemlichkeit für den Benutzer nicht zu beeinträchtigen, ist in weiterer Ausbildung dieser Ausführungsform vorgesehen, daß die Schiene an ihrer Oberseite im Bereich zwischen den in den Endbereichen der Schiene vorgesehenen Lautsprecheraussparungen eine Ausnehmung aufweist, in welche eine Schaumstoffleiste aus einem elastisch verformbaren Material, das weicher als das Hartschaummaterial ist und vorzugsweise aus dem Material des Kissenkörpers besteht, eingesetzt ist.

Nach einer weiteren Ausführungsform der Erfindung ist vorgesehen, daß die Schiene so ausgebildet ist, dass die Lautsprecher an der Schiene verstellbar angeordnet werden können. Diese Ausführungsform macht es möglich, die Lautsprecherposition individuell an die Kopfform des Benutzers anzupassen. Weiterhin ist es von Vorteil, wenn in der Schiene insbesondere schlitzförmige Durchgangsöffnungen für die erforderlichen Versorgungs- und/oder Übertragungskabel vorgesehen sind.

Eine weitere vorteilhafte Ausgestaltung der Erfindung weist einen insbesondere als Wechselbezug ausgebildeten Kissenbezug auf, der bei Bedarf problemlos gereinigt werden kann. In dem Kissenbezug sind dann zweckmäßigerweise an der Kissenunterseite nahe den einander gegenüberliegenden Querseiten Öffnungen vorgesehen, durch welche die Versorgungs- und/oder Übertragungskabel für die Lautsprecher geführt werden können. Die Anordnung von Öffnungen an beiden Querseiten und insbesondere den Eckbereichen des Kissens ermöglicht es dabei, die Kabel je nachdem, ob man auf der linken oder der rechten Seite schläft, in nicht störender Weise zu positionieren.

Hinsichtlich weiterer vorteilhafter Ausgestaltungen der Erfindung wird auf die Unteransprüche sowie die nachfolgende Beschreibung eines Ausführungsbeispiels unter Bezugnahme auf die beiliegende Zeichnung verwiesen. In der Zeichnung zeigt
- Figur 1: eine Ausführungsform eines erfindungsgemäßen Kissens in schematischer perspektivischer Ansicht,
- Figur 2: das Kissen aus Figur 1 in Unteransicht,
- Figur 3: die Schiene des Kissens in Vorderansicht und
- Figur 4: einen Kissenbezug für das Kissen in Unteransicht.

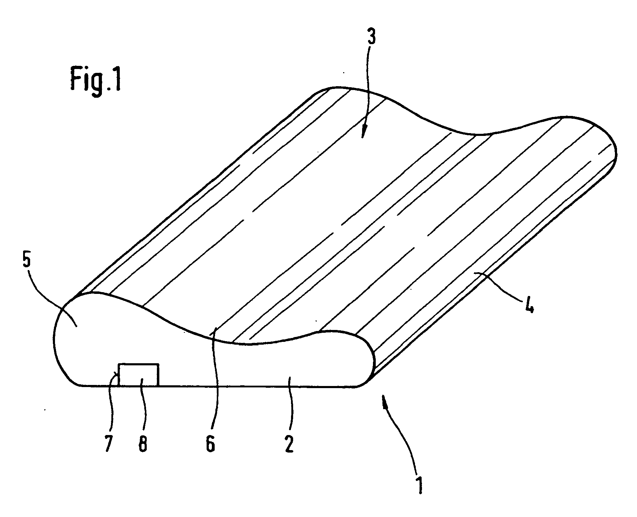

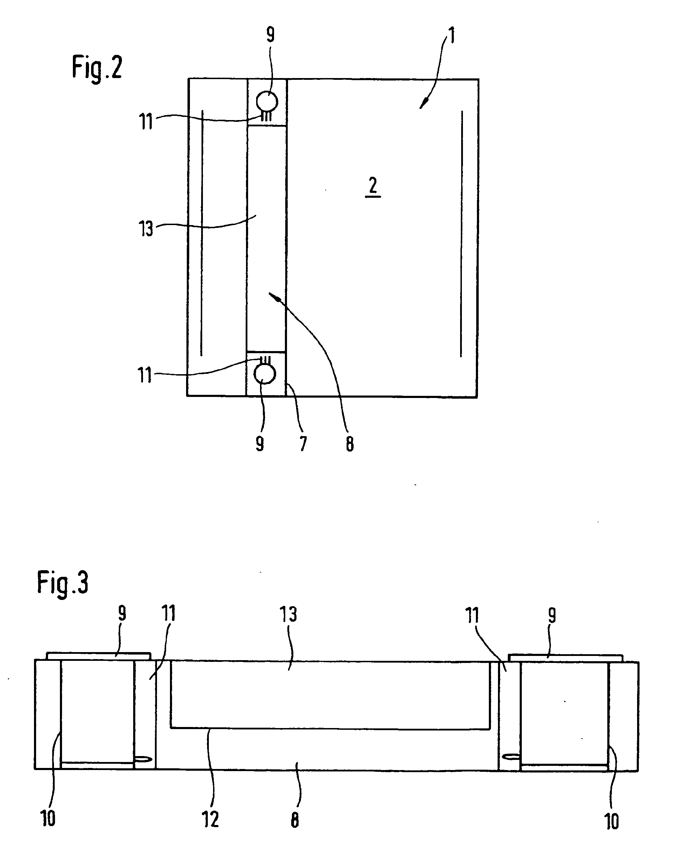

In den Figuren 1 und 2 ist ein musiktherapeutisches Kissen 1 gemäß der vorliegenden Erfindung dargestellt, das insbesondere zur Behandlung von Tinnitus-Erkrankungen eingesetzt werden kann. Das Kissen besitzt einen Kissenkörper 2 rechteckiger Grundform aus einem waschbaren elastischen Schaumstoffmaterial, das an seiner Oberseite eine orthopädisch geformte Auflagefläche 3 mit einer entlang einer Längsseite verlaufenden kleineren Nackenwulst 4, einer entlang der anderen Längsseite verlaufenden größeren Wulst 5 und einer dazwischen liegenden Mulde 6 aufweist.

Im Übergangsbereich zwischen der dicken Wulst 5 und der Mulde 6 ist an der Unterseite des Kissenkörpers 3 eine Ausnehmung 7 etwa rechteckigen Querschnitts vorgesehen, die sich über die gesamte Länge des Kissenkörpers 2 erstreckt. In diese Ausnehmung 7 ist eine Schiene 8 eingesetzt, in welcher ein Lautsprechersystem, das in dem vorliegenden Fall aus zwei Aktivlautsprechern 9 besteht, aufgenommen ist. Hierzu sind in den Bereichen der Schiene 8 jeweils Durchgangsöffnungen 10 vorgesehen, in welche die Lautsprecher 9 eingesetzt sind. Von diesen Durchgangsöffnungen 10 gehen ebenfalls durchgehende Kabelschlitze 11 ab, in welchen die Lautsprecheranschlüsse und die nicht näher dargestellten Versorgungskabel für die Lautsprecher 9 positioniert sind.

Die Schiene 8 besteht aus einem Hartschaummaterial, so daß sie eine ausreichende Stabilität besitzt und außerdem die Lautsprecher 9 ohne zusätzliche Befestigungsmittel wie beispielsweise Haftstreifen in den Durchgangsöffnungen 10 gehalten werden. Die Verwendung einer Schiene aus Schaumstoff hat weiterhin den Vorteil, daß die Schiene 8 bei entsprechender Dimensionierung in der Ausnehmung 7 des Kissenkörpers 2 von alleine hält und nicht durch Klebestreifen, Klettbänder oder dergleichen fixiert zu werden braucht, auch wenn solche zusätzlichen Befestigungsmittel der Sicherheit halber vorgesehen sein können.

Zwischen den Endbereichen, in denen die Durchgangsöffnungen 10 für die Lautsprecher 9 vorgesehen sind, ist in der Oberseite der Schiene 8 eine Ausnehmung 12 vorgesehen, in die eine Schaumstoffleiste 13 aus den gleichen Material, aus welchem auch der Kissenkörper 2 besteht, eingesetzt ist. Hierdurch wird erreicht, daß der Schlafkomfort durch das härtere Material der Schiene 8 nicht oder nur wenig beeinflußt wird.

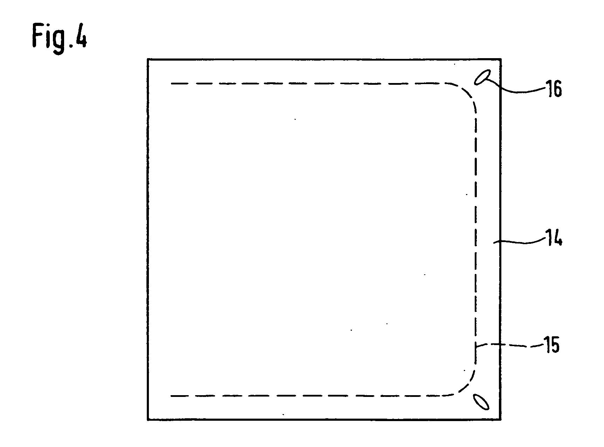

In Figur 4 ist ein Kissenbezug für das Kissen 1 dargestellt. Dieser Kissenbezug 14 besteht aus einem waschbaren Material und besitzt an seiner Kissenunterseite einen schematisch angedeuteten Reißverschluß 15, so daß er von dem Kissenkörper 12 abgezogen und auf diese Weise leicht gereinigt werden kann. In den nackenbereichsnahen Ecken besitzt der Kissenbezug 14 zwei Öffnungen 16, durch welche Anschlußkabel für die Lautsprecher 9 geführt werden können. Die Öffnungen 16 sind in beiden Eckenbereichen vorgesehen, so daß der Benutzer die Anschlußkabel wahlweise durch die eine oder andere Öffnung 16 führen kann, je nachdem, ob er beim Schlafen auf der linken oder rechten Körperseite liegt.

Erfindungsgemäß gestaltet sich die Reinigung des Kissenkörpers sehr einfach, da nach dem Abziehen des Kissenbezugs 14 die Lautsprecher 9 mit der Schiene 8 auf einfache Weise entfernt werden können.

## Patentansprüche

1. Kissen, insbesondere für den Einsatz im Rahmen von therapeutischen Maßnahmen, mit einem Kissenkörper (2) aus einem elastisch verformbaren Material, in dem mehrere Lautsprecher (9) positionierbar sind und der an seiner Oberseite eine Auflagefläche (3) bildet, **dadurch gekennzeichnet, daß** das Kissen eine Schiene (8) aus einem elastisch verformbaren Material enthält, die in eine korrespondierende Ausnehmung (7) an der Unterseite des Kissenkörpers (2) lösbar einsetzbar ist, wobei die Schiene (8) so ausgebildet ist, daß Lautsprecher (9) in der Schiene einsetzbar sind.

2. Kissen nach Anspruch 1, **dadurch gekennzeichnet, daß** die Schiene (8) Aussparungen (10) insbesondere in Form von Durchgangsöffnungen aufweist, in welche die Lautsprecher (9) einsetzbar sind.

3. Kissen nach Anspruch 2, **dadurch gekennzeichnet, daß** die Lautsprecheraussparungen (10) in den axialen Endbereichen der Schiene (8) vorgesehen sind.

4. Kissen nach einem der vorherigen Ansprüche, **dadurch gekennzeichnet, daß** die Schiene (8) zumindest größtenteils aus Hartschaum besteht.

5. Kissen nach den Ansprüchen 3 und 4, **dadurch gekennzeichnet, daß** die Schiene (8) an ihrer Oberseite im Bereich zwischen den Lautsprecheraussparungen (10) eine Ausnehmung (12) aufweist, in welche eine Schaumstoffleiste (13) aus einem elastisch verformbaren Material, das weicher als das Hartschaummaterial ist, eingesetzt ist.

6. Kissen nach einem der vorherigen Ansprüche, **dadurch gekennzeichnet, daß** die Schiene (8) so ausgebildet ist, daß die Lautsprecher (9) an der Schiene (8) verstellbar angeordnet werden können.

7. Kissen nach einem der vorherigen Ansprüche, **dadurch gekennzeichnet, daß** in der Schiene (8) insbesondere schlitzförmige Durchgangsöffnungen (11) für Kabel ausgebildet sind.

8. Kissen nach einem der vorherigen Ansprüche, **dadurch gekennzeichnet, daß** der Kissenkörper (2) orthopädisch geformt ist.

9. Kissen nach einem der vorherigen Ansprüche, **dadurch gekennzeichnet, daß** der Kissenkörper (2) eine Nackenwulst (4) besitzt und die Ausnehmung (7) für die Schiene (8) im Bereich dieser Nackenwulst (4) an der Kissenunterseite vorgesehen ist.

10. Kissen nach einem der vorherigen Ansprüche, **dadurch gekennzeichnet, daß** der Kissenkörper (2) aus einem Schaumstoffmaterial besteht.

11. Kissen nach Anspruch 10, **dadurch gekennzeichnet, daß** das Schaumstoffmaterial waschbar ist.

12. Kissen nach einem der vorherigen Ansprüche, **dadurch gekennzeichnet, daß** ein Kissenbezug (14) vorgesehen ist.

13. Kissen nach Anspruch 12, **dadurch gekennzeichnet, daß** der Kissenbezug (14) an der Kissenunterseite Öffnungen (16) nahe den gegenüberliegenden Querseiten aufweist, durch welche Versorgungs- und/oder Übertragungskabel für die Lautsprecher (9) geführt werden können.

## Claims

1. A cushion, in particular for use within the framework of therapeutic procedures, with a cushion body (2) made of an elastically deformable material, in which several loudspeakers (9) can be positioned, and which on its upper side forms a supporting surface (3), **characterised in that** the cushion includes a track (8) made of an elastically deformable material which can be inserted releaseably into a corresponding recess (7) on the lower side of the cushion body (2), the track (8) being designed such that loudspeakers (9) can be inserted into the track.

2. The cushion according to Claim 1, **characterised in that** the track (8) has cut-outs (10), in particular in the form of passageway openings, into which the loudspeakers (9) can be inserted.

3. The cushion according to Claim 2, **characterised in that** the loudspeaker cut-outs (10) are provided in the axial end regions of the track (8).

4. The cushion according to any of the preceding claims, **characterised in that** the track (8) is made at least largely of hard foam.

5. The cushion according to Claims 3 and 4, **characterised in that** on its upper side in the region between the loudspeaker cut-outs (10), the track (8) has a recess (12) into which a foam bar (13) made of an elastically deformable material which is softer than the hard foam material is inserted.

6. The cushion according to any of the preceding claims, **characterised in that** the track (8) is designed such that the loudspeakers (9) can be disposed adjustably on the track (8).

7. The cushion according to any of the preceding claims, **characterised in that** in the track (8), in particular slit-shaped passageway openings (11) are formed for cables.

8. The cushion according to any of the preceding claims, **characterised in that** the cushion body (2) is shaped orthopaedically.

9. The cushion according to any of the preceding claims, **characterised in that** the cushion body (2) has a bulge (4) for the neck, and the recess (7) for the track (8) is provided in the region of this bulge for the neck (4) on the lower side of the cushion.

10. The cushion according to any of the preceding claims, **characterised in that** the cushion body (2) is made of a foam material.

11. The cushion according to Claim 10, **characterised in that** the foam material is washable.

12. The cushion according to any of the preceding claims, **characterised in that** a cushion cover (14) is provided.

13. The cushion according to Claim 12, **characterised in that** the cushion cover (14) has openings (16) on the lower side of the cushion, close to the opposite transverse sides, through which supply and/or transmission cables for the loudspeakers (9) can be conveyed.

## Revendications

1. Oreiller, conçu en particulier pour être utilisé dans le cadre de mesures thérapeutiques, ayant un corps de l'oreiller (2) d'une matière élastique déformable, dans lequel sont positionnés plusieurs haut-parleur (9) et qui forme sur son côté supérieur une surface d'appui (3), **caractérisé en ce que**, l'oreiller contient un rail (8) d'une matière élastique déformable, qui peut être introduite de manière détachable dans une cannelure correspondante (7) du côté inférieur du corps (2) de l'oreiller, où le rail (8) est ainsi formé, de sorte que des haut-parleurs (9) puissent être positionnés dans le rail.

2. Oreiller selon la revendication 1, **caractérisé en ce que**, le rail (8) présente des dégagements (10), spécialement en forme d'orifices de passage, dans lesquels on peut positionner les haut-parleurs (9).

3. Oreiller selon la revendication 2, **caractérisé en ce que**, les dégagements (10) pour les haut-parleurs sont disposés dans les zones axiales d'extrémité du rail (8).

4. Oreiller selon l'une des revendications antérieures, **caractérisé en ce que**, le rail (8) est formé au moins dans la plus grande partie d'écume solide.

5. Oreiller selon les revendications 3 et 4, **caractérisé en ce que**, le rail (8) présente sur son côté supérieur dans la zone située entre les dégagements (10) pour haut-parleur une cannelure (12), où on introduit une règle de matériau spongieux (13), d'un matériau élastique déformable, plus mou que l'écume solide.

6. Oreiller selon l'une des revendications antérieures, **caractérisé en ce que**, le rail (8) est ainsi formé, de sorte que des haut-parleur (9) puissent être positionnés de manière mobile sur le rail (8).

7. Oreiller selon l'une des revendications antérieures, **caractérisé en ce que**, dans le rail (8) sont pratiqués des orifices de passage en forme de fentes (11) pour les câbles.

8. Oreiller selon l'une des revendications antérieures, **caractérisé en ce que**, le corps (2) de l'oreiller est formé de manière orthopédique.

9. Oreiller selon l'une des revendications antérieures, **caractérisé en ce que**, le corps (2) de l'oreiller présente un appui pour la nuque (4) et que la cannelure (7) pour le rail (8) est pourvue dans la zone du cet appui de la nuque (4) sur le côté inférieur de l'oreiller.

10. Oreiller selon l'une des revendications antérieures, **caractérisé en ce que**, le corps (2) de l'oreiller est formé d'un matériau spongieux.

11. Oreiller selon la revendication 10, **caractérisé en ce que**, le matériau spongieux est lavable.

12. Oreiller selon l'une des revendications antérieures, **caractérisé en ce que**, il est pourvu une taie (14) d'oreiller.

13. Oreiller selon la revendication 12, **caractérisé en ce que**, la taie (14) d'oreiller présente sur le côté inférieur de l'oreiller des ouvertures (16) près des parties latérales opposées, par où on peut amener les câbles d'alimentation et/ou de signal pour les haut-parleur (9).
